(19) Europäisches Patentamt
European Patent Office
Office européen des brevets

(11) **EP 3 827 806 A1**

(12) **EUROPEAN PATENT APPLICATION**

(43) Date of publication:
**02.06.2021 Bulletin 2021/22**

(51) Int Cl.:
**A61K 8/02** *(2006.01)*    **A61K 8/73** *(2006.01)*
**A61Q 19/00** *(2006.01)*

(21) Application number: **19212217.4**

(22) Date of filing: **28.11.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(71) Applicant: **BASF SE**
**67056 Ludwigshafen am Rhein (DE)**

(72) Inventors:
• **Mueller, Jan Ole.**
**67056 Ludwigshafen am Rhein (DE)**

• **Oschmann, Bernd Dieter.**
**67056 Ludwigshafen am Rhein (DE)**
• **Witteler, Helmut.**
**67056 Ludwigshafen am Rhein (DE)**
• **Beziau, Antoine Maxime Charles Joseph.**
**67056 Ludwigshafen am Rhein (DE)**
• **Kuenne, Holger.**
**67056 Ludwigshafen am Rhein (DE)**

(74) Representative: **BASF IP Association**
**BASF SE**
**G-FLP-C006**
**67056 Ludwigshafen (DE)**

(54) **BIODEGRADABLE BEADS WITH DYES AND PIGMENTS**

(57)    The present invention relates to biodegradable spherical beads comprising amorphous cellulose as carrier and incorporated dyes and/or pigments, preferably these beads are also comprising cosmetic oils.

Depending on the properties and composition of the beads, they can be used for an easy and convenient application of cosmetic emollients or as peeling agent and cleaning agent in the skincare field.

EP 3 827 806 A1

**Description**

The present invention relates to biodegradable spherical beads comprising amorphous cellulose as carrier and incorporated dyes and/or pigments.

Dyes and pigments are often used in cosmetics and personal care products. Especially decorative cosmetics comprise many products with dyes and pigents as a main ingredient due to their focus on beauty and appearance.

The major use is based on their ability to mask impurities of the skin. Being colored these particles provide a splendid look to cosmetic compositions and tempt users to feel good when using exactly this composition. Sometimes dyes or pigments are incorporated to cover unwanted grey, brown or impure looking adjuvants in cosmetic compositions.

In PCT-application WO2010 019164 a gel system comprising translucent microparticles, titanium dioxide and color pigments within a fractal network of nanoparticles for blurring fine lines wrinkles and pores to improve skin appearance is disclosed.

Elastomeric macroparticles forming the gel network are made of cellulose and cellulose derivatives among many other suitable polymers such as crosslinked silicone polymers, (meth)acrylate polymers, ethylene and propylene polymers, natural and synthetic rubbers. Many of these polymers are not readily biodegradable and fall under the definition of microplastics.

Microplastics are defined as synthetic polymers below 5 mm in size which are not biodegradable. Microplastic particles are extremely harmful to marine life not only because of their morphological properties but also due to the adsorption of environmental toxins on their large surface area by physico-chemical adhesion of the plastic material.

In the past years these microplastic particles have been added to cosmetics and personal care products, but have meanwhile been replaced by natural material, such as for example ground nut shells, apricot kernels, minerals, waxes or biodegradable polymers. The cosmetic market is looking for further easily biodegradable and environmentally compatible polymers, so that the use of cellulose was investigated in more detail.

It was one object of the invention to provide colored beads for cosmetic and personal care products which do not have a negative impact on our environment and could substitute hazardous microplastic beads.

Beads having diameters of 0.1 $\mu$m up to a few millimeters comprising cellulose as forming matrix have been used in many fields and advanced applications. Adsorbing or embedding different active components these beads are used for filtering, waste water treatment, chromatography or in pharmaceutic and cosmetic compositions.

Cellulose containing microspheres comprising magnetic $Fe_3O_4$ iron oxide nanoparticles for targeting protein delivery and release have been prepared by sol-gel-transition (Xiaogang Luo et al., Journal of Materials Chemistry 2009, 19, p. 3538-3545, First published as an Advance Article on the web 17th April 2009).

Xiaogang Luo and Lina Zhang have investigated the adsorption capacity of negatively or positively charged organic dyes with porous cellulose particles containing magnetic $Fe_2O_3$ nanoparticles and activated coal for wastewater treatment. By dropping the cellulose solution comprising $Fe_2O_3$ - nanoparticles and activated coal powder dispersed therein into a sodium chloride bath spherical beads with a mean diameter of 2.2 mm were successfully manufactured. These beads were spherical and dark brown due to the presence of activated coal. ("High effective adsorption of organic dyes on magnetic cellulose beads entrapping activated carbon", Journal of Hazardous Materials, 171, (2009), p. 340-347.)

Taro Omura et al. have manufactured cellulose particles comprising a fluorescent substance in a spongy structure ("Encapsulation of Either Hydrophilic or Hydrophobic Substances in Spongy Cellulose Particles"; American Chemical Society - ACS Appl. Mater. Interfaces 2017, 9, p. 944-949).

Storing these beads at room temperature they collapse because the solvent inside them evaporates. The beads are dried with supercritical carbondioxide to develop the spongy structure which enables the absorption of a dye in toluol or water.

The manufacturing process includes the dissolution of cellulose in ionic liquids and DMF. For personal care, cosmetic and pharmaceutical use ionic liquids such as imidazolium salts and many solvents for dissolution of cellulose such as dimethylformamid must be avoided due to their missing biocompatibility. Ionic liquids are difficult to be washed out and expensive to be recycled.

For cosmetic use it is advantageous if the particles are not only colored but are a vehicle to apply cosmetic especially oily ingredients.

Hence it was a further object that the colored beads of the invention could carry cosmetic ingredients and enable the user to easily apply these ingredients on the skin.

**Detailed description of the invention**

These objects were solved by providing spherical beads with a diameter D(4,3) of 1 - 4000 $\mu$m comprising
1 to 99.9 % by weight of amorphous cellulose,
0.01 to 20 % by weight of dyes and/or pigments,
characterized in that the weight ratio of dyes and pigments to cellulose is between 0.001/99.999 and 20/80.

The dyes or pigments are incorporated into the cellulose matrix and not only adsorbed on the surface of the cellulose beads.

The beads of the invention have a specific physical stability as they are stable at room temperature outside a dispersion medium but collapse when they are subjected between two parallel plates administrating a force of at least 0.05 N. This stability enables storage of the beads at room temperature without the use of dispersion media, an easy handling of the beads for further processing as well as transport and possibilities to use them in in-situ-preparations of the customer/end-user.

Two types of colored beads - one species comprising oil components and another comprising a high amount of amorphous cellulose without additional oils - are provided depending on the respective use.

Beads comprising oils can rupture by applying a force with the user's hand or fingers to spread the incorporated cosmetic or oily ingredients on the skin, mucosa or hair. After release of the entrapped ingredients the latter could easily and evenly be distributed on the skin and result in a pleasant and soft skin feeling.

The beads comprising a high amount of cellulose and a low amount of oils or no oily components could be used as a peeling or scrubbing agent because their abrasive properties can be adjusted by the amount of cellulose and the type and amount of incorporated agents.

Soft beads comprise a lower amount of cellulose and a higher amount of oils or emollients while harder beads with a higher concentration of cellulose and lower loading capacity have better abrasive properties.

Depending on the use of the beads, for example the distribution of emollients for hydration of the skin and a smooth skin feeling or the use as peeling agent with abrasive properties for cleaning the skin the composition of the beads can be adjusted.

Soft beads could contain up to 98 weight % of an oily ingredients such as cosmetic oils or emollients based on the weight of the beads. Beads with abrasive properties are comprising at least 50 % by weight, preferably at least 90 % by weight, more preferably at least 95 % by weight and most preferably at least 98 % by weight of amorphous cellulose based on the weight of the beads.

Colored beads which could be used as a peeling agent with abrasive properties are comprising

97 - 99.9 % by weight of amorphous cellulose and

0.1 - 3 % by weight of a dye or pigment

based on the weight of the beads and more preferable they comprise

97.5 - 99.5 % by weight of amorphous cellulose and

0.5 - 2.5 % by weight of a dye or pigment based on the weight of the beads.

Colored soft beads for the application of cosmetic oils and emollients are comprising

1 - 20 % by weight amorphous cellulose and

0.1 - 98 % by weight of an oily ingredient and

0.01 to 10 % by weight of a dye or pigment

based on the weight of the beads and

more preferably they comprise

1 - 10 % by weight amorphous cellulose and

80 - 98 % by weight of an oily ingredient and

0.05 to 10 % by weight of a dye or pigment based on the weight of the beads.

Beads comprising further ingredients might as well contain a medium amount of cellulose and medium amount of an oily ingredient such as beads comprising

55 - 75 % by weight amorphous cellulose and

0.1 - 43 % by weight of an oily ingredient and

0.01 to 10 % by weight of a dye or pigment

based on the weight of the beads,

preferably beads comprising

55 - 65 % by weight amorphous cellulose and

5 - 43 % by weight of an oily ingredient and

0.01 to 10 % by weight of a dye or pigment

based on the weight of the beads,

or

70 - 75 % by weight amorphous cellulose and

5 - 29 % by weight of an oily ingredient and

0.01 to 20 % by weight of a dye or pigment

based on the weight of the beads,

more preferably beads comprising

70 - 75 % by weight amorphous cellulose and

20 - 29 % by weight of an oily ingredient and

0.01 to 5 % by weight of a dye or pigment
based on the weight of the beads.

Also possible are beads containing a lower amount of cellulose and medium amount of an oily ingredient such as beads comprising

21 - 54 % by weight amorphous cellulose and
20 - 78 % by weight of an oily ingredient and
0.01 to 10 % by weight of a dye or pigment
based on the weight of the beads,

Measurement of the collapsing force:

Description of texture analyzing method:

The diameter (D) of a bead (A) is determined by use of an optical microscope. The feature "diameter (D)" and any particle or beads sizes disclosed in the current invention will always mean D(4,3), which describes the volume-weighted average mean of all particles.

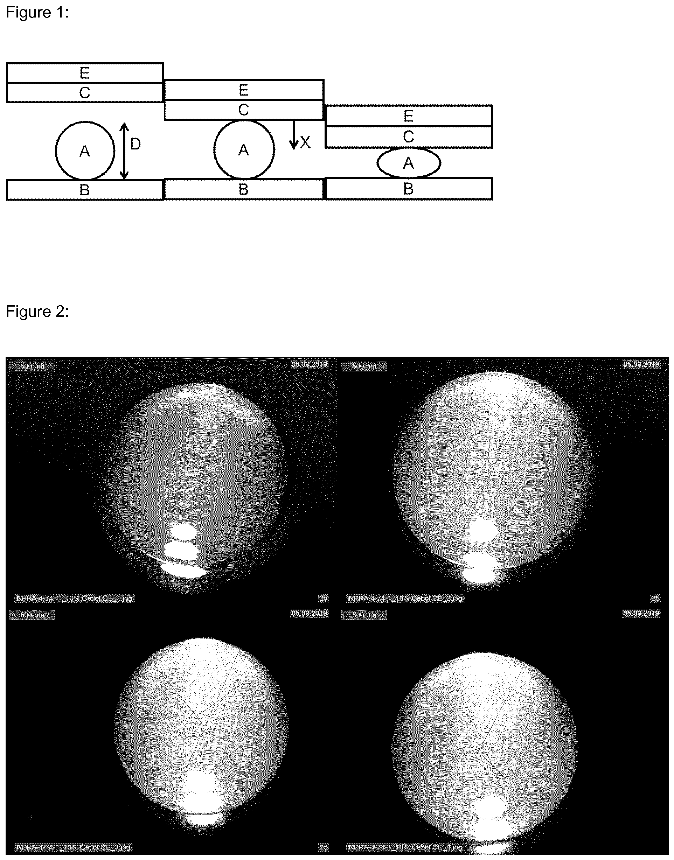

The deformation curve of the bead is measured with a Texture Analyzer TA.XT by WINOPAL (Forschungsbedarf GmbH, Elze, Germany). For this measurement (see figure 1), a single bead (A) of a defined diameter (D) is placed on a flat surface (B) under the centre of a flat stamp (C) of 32 mm diameter, which is parallel to surface (B) and it is equipped with a pressure sensor (E). Stamp (C) is lowered with a speed of 2 mm/s until the sensor (E) registers a force of 0.05 N (figure 1). After that, the speed of the stamp is reduced to 1 mm/s and the recording of the compression force (F) as measured by (E) starts. At the same time, the displacement (X) of (C) is recorded. For data evaluation, (F) is plotted versus (X). The force (F50) that is required to compress a bead to 50% of its initial diameter (X = 0.5 x D) is retrieved from the recorded data.

The beads are characterized in that their deformation between two parallel plates obeys to the rules

$F_{50}$ < 0.03 x D -14.95 for beads of D(4,3) of 500 to 1500 $\mu$m and
$F_{50}$ < 0.12 x D -149.95 for beads of D(4,3) of 1500 to 3000 $\mu$m

with $F_{50}$ being the force measured in N necessary to compress the bead to 50% of its original diameter D measured in $\mu$m and

wherein $F_{50}$ is larger than 0.05 N for beads containing more than 50 % by weight of oily ingredients, preferably more than 80 % by weight of oily ingredients based on the weight of the beads,

$F_{50}$ < 55 N for beads of D(4,3) of 500 to 1500 $\mu$m and
$F_{50}$ > 30 N for beads of D(4,3) of 500 to 1500 $\mu$m

with $F_{50}$ being the force measured in N necessary to compress the bead to 50% of its original diameter D measured in $\mu$m for beads containing 10 to 50 % by weight based of oily ingredients based on the weight of the beads.

$F_{50}$ > 55 N for beads of D(4,3) of 500 to 1500 $\mu$m
with $F_{50}$ being the force measured in N necessary to compress the bead to 50% of its original diameter D measured in $\mu$m for beads containing no oily ingredients.

**Beads Size**

Measurement of the beads diameter can be conducted by laser diffraction and by light microscopy

Determination of particle size distribution by laser diffraction:

Particle Size Distribution (PSD) was determined by laser diffraction using a Malvern Mastersizer 2000 according to European norm ISO 13320:2009 EN. The data were treated according to the Mie-Theory by software using a "universal model" provided by Malvern Instruments. Important parameters are in particular the following values: D(v 0,5), D(v 0,9), D(v 0,1), D(3,2) and D(4,3), where D(v 0,5), D(v 0,9), D(v 0,1), D(3,2) and D(4,3) are as defined herein. In the context of particle size, the D(0,9) or D(v 0,9) value of particle size indicates that 90 vol.-% of the particles have a hydrodynamic diameter smaller than this value. In the context of particle size, the volume median particle diameter D(0,5) or D(v 0,5) value, respectively, means that 50 vol.-% of the particles have a diameter which is above the value cited and 50 vol.-% of the particles have a diameter which is below the value cited. In the context of particle size, the D(0,1) or D(v 0,1) value indicates that 10 volume % of the particles have a hydrodynamic diameter below the value cited. The D(3,2) describes the surface-weighted average mean of all particles, while the D(4,3) describes the volume-weighted average mean of all particles.

Determination of particle size distribution by light microscopy:

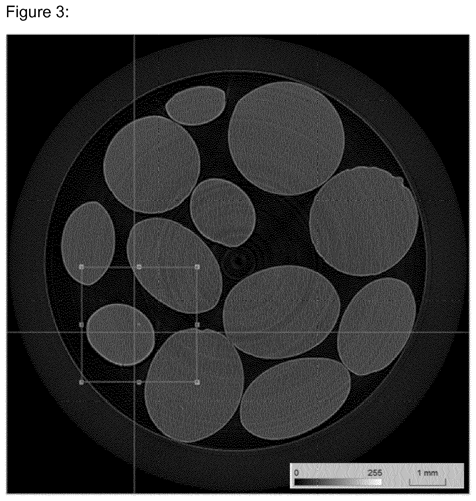

Particle Size Distribution (PSD) was determined by optical light microscopy (Leica Z16 APO zoom-system + Leica DFC420 digital color camera and Leica KL 1500 LCD light source) and manual measurement (double determination) of the diameter of 4 - 10 representative particles using the software Leica Application Suite (LAS) Core (see figure 3: beads size determination by light microscopy (beads of example 3.16)).
The volume based average particle size D(4,3) was calculated according to formula I:

## Formula I

$$D(4,3) = \frac{\sum_1^n Di^4}{\sum_1^n Di^3}$$

The beads of the invention are small solid or semi-solid objects with mean diameters D(4,3) of 1 to 4000 $\mu$m, preferably 10 to 3000 $\mu$m, more preferably 100 to 2500 $\mu$m, most preferably 500 to 2000 $\mu$m determined by light microscopy.
Different size ranges are achieved depending on the respective way of manufacturing, so that specific ranges between 10 and 100 $\mu$m, or between 100 and 500 $\mu$m, or between 500 and 1500 $\mu$m, or between 1000 and 2500 $\mu$m are preferred.
The beads of this invention are "spherical", which means that the ratio between length and width of the beads is between 1 and 2, preferably between 1 and 1.5 and more preferably between 1 and 1.2.

**Amount of water**

While beads of the prior art comprise a certain amount of water due to their manufacturing method the amount of water in the inventive beads is very low. It is less than 10 % by weight, preferable less than 5 % by weight, more preferably less than 3 % and most preferably less than 1 % by weight of water based on the weight of the beads. The water content has an influence of the physical and chemical stability of the beads. The lower the amount of water the better is the loading capacity of the oils.

Determination of water content in cellulose particles

A sample of the cellulose beads is weighted into a glass ampoule and introduced in a 120°C preheated oven (874 Oven Sample Processor, Methrom). The water vapor is transferred into the previously dry-titrated Coulometer (852 Tritrando, Methrom) by a stream of dry nitrogen. The content of water is determined coulometrically by bipotentiometric indication. The minimum time of the measurement is 300 s. A drifting is determined at the beginning of the measurement (startdrift), the measurement is stopped when the drift is equal to a startdrift plus 15 $\mu$g/min, but measurement time is at least 300 s. The relative standard deviation of the determination is below 1%.

**Porosity**

The porosity of the particles has not been investigated further as BET - and CT-measurements have not shown any pores on the surface and inside the beads (see figure 3). The beads are completely homogeneous inside, a porosity cannot be detected. Figure 3 shows A Micro-CT measurement of cellulose beads of example 2.1 of the cross section of particles.
A BET-measurement (Instrument: Gemini VII, Micromeritics, Nitrogen absorption for 24 h at 23°C with a relative pressure range p/p$_0$ of 0.05 to 0.3)) of example 2.1 has given a BET-surface of 0.1756 m$^2$/g with a BET-constant of 7.68.
It is clearly shown, that no pores of a detectable size range can be discovered. This low value for the BET-surface confirms the observation of the $\mu$-CT measurements, where no pores in $\mu$m size range could be observed. The absence of pores in the range of the visible light wavelength would also explain the translucence of the beads. As the beads do not have a porous structure the loading with the dyes and pigments needs to be considered during the processing of the beads before a final drying step. Consequently, loaded beads contain ingredients which are incorporated in the matrix of the beads.

**Translucence**

Preferably the spherical beads which comprise soluble dyes but no pigments are translucent. Only in case that the cellulose is in an amorphous state and no crystallinity can be detected a translucence is possible. "Translucent" means that the beads allow light to pass through partially or diffusely. They have a glassy, (semi)transparent look and they are permitting light to pass through but diffusing it so that objects on the other side cannot be clearly visible.

The translucence is only possible if the complete cellulose of the bead is in an amorphous state.
The beads disclosed in the European patent EP2190882 have a degree of crystallinity of 15 - 35% due to the applied solvent system and can consequently not be translucent.

The beads disclosed in US application US 2004/0131690 comprise flocculated microcrystalline cellulose. They are not translucent because of the presence of cellulose crystallites due to their way of manufacturing. Oil-filled beads with flocculated cellulose are manufactured by (a) preparing an aqueous dispersion comprising colloidal microcrystalline cellulose; (b) preparing an oil phase comprising an oil; (c) combining the aqueous dispersion and the oil phase to form an emulsion; and (d) adding portions of the emulsion to a setting bath comprising water and at least one flocculating agent selected from a salt, an organic solvent, a pH modifier or a cationic material. Step a) comprises the dispersion of colloidal microcrystalline cellulose and this colloidal microcrystalline cellulose is then flocculated by the flocculating agent, following that the structure of the beads is porous, not homogenous inside and not translucent.

Beads as manufactured according to Taro Omura et al. - ACS Appl. Mater. Interfaces 2017, 9, p. 944-949 - collapse and become opaque after evaporation of the solvent 1-butanol.

Determination of transparency/translucence of cellulose particles with a UV-VIS-spectrophotometer:

Transparent in the sense of this invention is a cellulose bead when it appears brighter than a reference material that exhibits a transparency for visible light of 0.6%. The inventive cellulose beads and the reference material are observed and compared in direct light. "Direct light" means that the specimen and the reference material are placed between the eye of the observer and the source of light. "Light" is visible light or monochromatic light from the visible spectrum.

As reference material for transparency, a flat material with limited transparency is used, here a non-colored stationary paper of a thickness of 80 g/m$^2$. The transparency of the reference material is measured with a UV-VIS-spectrophotometer. The reference material is put into a cuvette perpendicular to the light beam of the spectrophotometer.

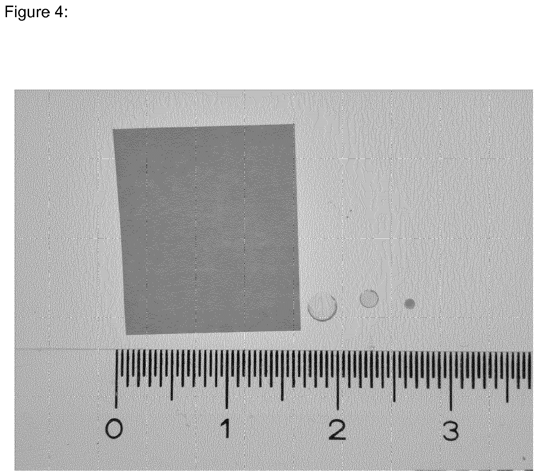

Measurement of the transparency of the reference material in figure 4:
The transparency of the reference material (stationary paper of 80 g/m$^2$) is 0.4% at 350 nm, 0.5% at 450 nm and 0.6% at 600 nm. Measured with a spectrophotometer is DR 6000 by Hach Lange GmbH, Düsseldorf, Germany.

Figure 4: Photography (viewed in direct light) of - from left to right - reference material (stationary paper 80 g/m$^2$), inventive material 1 (beads of example 3.15) comparison material 2 (beads of example 2.2) and comparison material cellulose pellet (Vivapur CS 800 S by Rettenmeier, Rosenberg). Samples are placed on a light box (Color Control Professional by Just Normlicht, Weilheim, Germany). Photo is taken with a camera SP-590US by Olympus.

**Content of oily ingredients**

In one embodiment of the invention the beads comprise a high amount of oily ingredients while prior art beads have much lower loading capacities. The beads of the current invention could comprise 0.1 to 98 % by weight of an oily ingredient. For the application of oils and emollients on the skin the colored beads preferably comprise at least 20 % by weight, preferably at least 40 % by weight and more preferably at least 80 % by weight and most preferably at least 95 % by weight of the oily ingredient based on the weight of the beads.

It is surprising that despite of the high load of oily ingredient, preferably emollient, the beads still have sufficient stability without storage in a dispersion medium.

Determination of oily ingredients content (*AC*) in cellulose particles:

Calculation according to formula II.

Equipment: 3 l beaker with PTFE half-moon-blade stirrer and three diaphragm pump SIMDOS 02 from KNF.

The beaker was charged with 2000 mL of ethanol. With a rate of 3 mL/min, 193.6 g of cellulose solution in TBAAc/MeCN (2.5 wt.% MCC) were dropped from a height of 0.5 cm to the stirred ethanol. The stirrer was stopped after complete addition of the feed. The obtained cellulose beads sink to the bottom of the breaker immediately upon addition to the ethanol. 1h after completion of the feed, the cellulose beads were isolated by filtration. The cellulose beads were suspended in 2000 mL fresh ethanol for 1 h. This washing step was repeated one more time. Three samples of the ethanol-containing beads were dried in a vacuum oven for 2 h at 120°C. The measured solid content (*SC*) was 3.8 %.

69.1 g of isolated beads (beads swollen with solvent = $B_{solv}$) were added to a mixture of 251.3 g of Cetiol® OE and 249.8 g of ethanol in a 1 L glass beaker. After 1 h the cellulose beads were isolated by filtration and added 502.6 g of fresh Cetiol OE. This step was repeated with 507.1 g of Cetiol® OE. The open vial with Cetiol OE and cellulose beads were stored for 2 days in a vacuum cabinet at 50 mbar and ambient temperature. The cellulose beads were isolated by filtration. The weight of the isolated beads was 62.4 g (beads loaded with active = $B_{active}$). These beads were added to 500.2 g fresh Cetiol®. The specific example is calculated according to formula III.

Formula II

$$\frac{B_{active} - (B_{solv} \; x \; SC\%)}{B_{active}} = AC\%$$

Formula III:

$$\frac{62.4 - (69.1 \; x \; 3.8\%)}{62.4} = 95.8\%$$

The beads of the present invention are stable under various conditions. At dry storage under air at ambient conditions the beads maintain there spherical form without significant release of oily ingredient. Even upon storage for at least 9 months the particles are stable without disintegration and no changes to the initial appearance. Storage in aqueous environment is also feasible without disintegration of the beads. The particles maintain their spherical form as well as a significant amount of oily ingredient. The leakage of oily ingredient was not quantified.

**Cellulose**

The beads of the current invention are characterized in that they comprise 1 to 99.9 % by weight of amorphous cellulose based on the weight of the beads.

Cellulose is a polysaccharide consisting of long unbranched chains of β-1,4-linked D-glucopyranose units. As described by Parks et al. (Biotechnology for Biofuels, 2010, 3:10 p. 3 to 10: Cellulose crystallinity index: Measurement techniques and their impact on interpreting cellulase performance. Sunkyu Park, John O. Baker, Michael E. Himmel, Philip A. Parilla and David K. Johnson) there are four types of cellulose with different crystallinity index.

For the invention different structures of cellulose are suitable, such as cellulose type I, a cellulose structure present in plants which is not changed by the recovery or cellulose type II, a thermodynamic more stable structure, which is prepared by dissolving type I cellulose and regenerating the cellulose.

Thus for the manufacturing of the beads various types of cellulose independent of their degree of crystallinity can be used as the cellulose is completely dissolved in a suitable solvent during the manufacturing process. The amount of lignin in the used cellulose should not be high due to its poor solubility, but commercially available cellulose types for cosmetic or pharmaceutical use can be used. Microcrystalline cellulose is preferred due to its purity and relatively low molecular weight.

The amount of cellulose must be adapted according to the intended use. More solid beads with higher peeling and scrubbing properties have a higher amount of cellulose, more softer beads with oily ingredients used to apply the emollients on the skin comprise a low amount of cellulose.

Solid beads have more abrasive properties while the soft beads are advantageous for the application of cosmetic oils.

Based on the application field the beads may comprise 95 to 99.9 % by weight, preferably 97 to 99.9 % by weight of amorphous cellulose for the solid version, 1 to 20 % by weight, preferably 1 to 10 % by weight, more preferably 1 to 6 % by weight of amorphous cellulose for the soft version and 55 to 75 % by weight of amorphous cellulose, specifically 55 to 65 % by weight or 70 to 75 % by weight for the medium version. All percentages based on the weight of the beads.

In the prefered embodiment of the invention the beads do not comprise cellulose derivatives. Biodegradability of the beads decreases if derivatized cellulose is used. This means that the cellulose which is used for manufacturing the beads is not functionalized by etherification, esterification, oxidation, grafting or any other derivatization because of the superior compatibility of pure cellulose in cosmetic or pharmaceutic applications and due to the biodegradability of the beads.

**Dyes and pigments**

Suitable dyes and pigments are any substances suitable and approved for cosmetic purposes. For example, the dyes listed in the publication "Kosmetische Färbemittel" of the Farbstoffkommission der Deutschen Forschungsgemeinschaft, Verlag Chemie, Weinheim, 1984, pages 81 to 106 and the dyes and pigments as sold as Puricolor®-types by BTC Europe GmbH, Monheim am Rhein, Germany and available byKoelcolour, Mumbai, India such as Colours for Cosmetic and Personal Care comprising:

Inorganic Pigments: Ultramarine Blue, Ultramarine Violet, Ultramarine Red / Pink, Mica, Chromium Oxide Green, Chromium Hydroxide Green, Iron Oxide Red, Iron Oxide Yellow, Iron Oxide Black, Iron Blue, Talc, Manganese Violet, Titanium Dioxide.

Organic Pigments: Brilliant Lake Red R (Ca Lake), Lake Phloxine, Lithol Rubine B (Ca Lake), Lithol Rubine B (Ba Lake), Lithol Rubine B (Na Salt), Deep Maroon (Ca Lake), Lake of Acid Fuchsine (Al Lake), Lake of Tetra Bromo Fluroscein, Lake of Phloxine B, Lake of Acid Orange [Al Lake], Lake Quinoline Yellow W. S., Lake Allura Red (Al Lake), Lake Tartrazine (Al Lake), Lake Sunset Yellow [Al Lake], Lake Brilliant Blue (Al Lake), Lake Erythrosine, Lake Ponceau SX [Al Lake], Lake Indigo Carmine, Lake of Quinoline Yellow W. S., Lake of Indigo Carmine, Lake of Carmoisine [Al Lake], Lake of Ponceau 4R, Lake Patent Blue V, Lake Black PN, Lake of Allura Red (Al Lake), Lake of Tartrazine (Al Lake), Lake of Sunset Yellow [Al Lake], Lake of Brilliant Blue (Al Lake), Lake of Erythrosine

Oil Soluble Colors: Alizarine Cyanine Green, Ponceau G, Uranine, Tetra Bromo Fluroscein, Tetra Bromo Fluroscein (W), Phloxine B, Quinoline Yellow S. S., Alizarine Purple, Quinizarine Green

Water Soluble Colors: Acid Orange 7, Acid Fuchsine, Acid Fuchsine, Phloxine B [Na Salt], Eosine [Na Salt], Quinoline Yellow W. S., Alizarine Purple Ext, Uranine (Na Salt), Fast Green FCF, Ponceau SX, Sunset Yellow, E-110, Allura Red, E-129, Tartrazine, E-102, Brilliant Blue, E-133, Erythrosine, E-127, Indigo Carmine, E-132, Ponceau 4R, E-124, Acid Red 52, Carmoisine, E-122, Amaranth, E-123, Brown HT, Black PN. Green S, Patent Blue V, Tartrazine, Quinolline Yellow, Erythrosine, Allura Red, Brilliant Blue.

The prefered dyes and pigments are Puricolor®-types by BTC Europe GmbH, Monheim am Rhein, Germany) and dyes including cochineal red A (C.I. 16255), patent blue V (C.I. 42051), indigotin (C.I. 73015), chlorophyllin (C.I. 75810), quinoline yellow (C.I. 47005), indanthrene blue RS (C.I. 69800), and madder lake (C.I. 58000). Luminol may also be present as a luminescent dye.

More preferred dyes are soluble dyes of the tradename Puricolor® available by BTC Europe selected from the group consisting of Puricolor® Red ARE33 FDA (Acid Red 33 (17200)),

Puricolor® Yellow AYE23 FDA (Acid Yellow 23 (CI 19140)), Puricolor® Green SGR7 FDA (Solvent Green7 (CI 59040)), Puricolor® Red FRE1 FDA (Food Red 1 (CI 14700)), Puricolor® Green U3 FDA (Food Green 3 (CI 42053)), Puricolor® Yellow FYE13 (Food Yellow 13 (CI 47005)).

Preferred pigments are selected from the group consisting of chlorophyll, titanium dioxide, zinc oxide, iron oxides such as red iron oxide, yellow iron oxide, brown iron oxide and black iron oxide; chromium oxides, chromium hydroxides, ultramarine blue, indigo pigments, manganese ammonium pyrophosphates, manganese violet, cerium oxide, copper phtalocyanines and mixtures thereof.

Due to their tolerability iron oxides are the preferred pigments for the beads of the current invention.

Prior art documents disclose cellulose beads for cleaning wastewater which often comprise activated coal. As the beads of the current invention are used in the cosmetic field they do not contain activated coal.

These dyes and/or pigments are normally used in a weight ratio of dyes and pigments to cellulose between 0.001/99.99 to 20/80, preferably 0.001/99.999 and 10/90, more preferably 0.01/99.99 to 5/95, even more preferably 0.1/99.9 to 2/98, and most preferably 0.5/99.5 to 1.5/98.5.

**Oily Ingredients**

Another object of the invention was the delivery of emollients to the skin in form of easily processible solid beads with a high loading and increased storage stability.

The oily ingredients incorporated in the beads can be any lipophilic ingredient which is suitable for dissolving dyes or dispersing dyes and/or pigments. Preferably they are selected from the group consisting of emollients, oil components, superfatting agents, waxes and mixtures thereof, most preferred are emollients.

The beads of the current invention could comprise 0.1 to 98 % by weight of an oily ingredient.

For the application of oils and emollients on the skin the colored beads preferably comprise at at least 20 % by weight, preferably at least 40 % by weight and more preferably at least 80 % by weight and most preferably at least 95 % by weight of the oily ingredient based on the weight of the beads.

In order to achieve a higher stability - depending on the incorporation of the beads in certain cosmetic compositions - the beads comprising a medium amount of oily ingredients with 0.1 to 43 % by weight of the oily ingredient will preferably have 5 to 43 % by weight, with preferred ranges of 5 to 29 and 20 to 29 % by weight of the oily ingredient based on the weight of the beads.

The incorporated oily ingredients have a molecular weight of more than 50 g/mol, preferably more than 100 g/mol. Incorporation of ingredients with lower molecular weight has shown to have limited use in cosmetic formulations.

The colored beads comprising these oily ingredients were easy to handle and stable enough for the incorporation in personal care compositions during the shelflife of cosmetic compositions. They broke down and released the incorporated emollient upon distribution or application of the cosmetic composition on the skin.

**Emollients**

In a prefered embodiment of the invention the incorporated oily ingredients are emollients. These are selected from the group consisting of fatty acid esters, esters of C6-C28 fatty acids and C6-C28 fatty alcohols, glyceryl esters, fatty acid ester ethoxylates, alkyl ethoxylates, C12-C28 fatty alcohols, C12-C28 fatty acids, Guerbet esters, Guerbet alcohols and Guerbet acids, saturated alkanes, C12-C28 fatty alcohol ethers, vegetable oils, natural essential oils, mineral oil, parafinum liquidum, petrolatum, isoparaffins, preferably the emollients are selected from the group consisting of dibutyl adipate (Cetiol® B), phenethyl benzoate, coco-caprylate (Cetiol® C5), coco-caprylate/caprate (Cetiol® LC, Cetiol® C5, Cetiol® C 5C), dicaprylyl Carbonate (Cetiol® CC), propylheptyl caprylate (Cetiol® Sensoft), caprylyl caprylate/caprate (Cetiol® RLF), myristyl myristate (Cetiol® MM), PEG-7 caprylic/capric glycerides (Cetiol® HE810), isononyl isononanoate (Cetiol® ININ) ; capric glycerides, coco-glycerides (Myritol® 331), capryl/caprin-triglyceride (Myritol® 312), capryl/caprin-triglyceride (Myritol® 318), C12-15 alkyl benzoate (Cetiol® AB), PPG-3 benzyl ether myristate, C12-13 alkyl lactate, isodecyl salicylate, alkyl malate, isoamyl laurate, propylheptyl caprylate, butyloctyl salicylate, polycrylene, dicaprylyl carbonate (Cetiol® CC), dicaprylyl ether (Cetiol® OE), 2-octyldodecylmyristate, isohexadecane, dimethyl capramide, squalene, isopropyl isostearate, isostearyl isostearate, decyl oleate (Cetiol® V), oleyl erucate (Cetiol® J 600), ethylhexyl stearate (Cetiol® 868); cetearyl ethylhexanoate (Luvitol® EHO), octyldodecanol (Eutanol® G), hexyldecanol (Eutanol® G16), volatile linear C8 to C16 alkanes, C10 to C15 alkanes, C11-C13 alkanes (Cetiol® Ultimate), C13-15 alkanes, C15-19 alkanes, C17-23 alkanes, isododecane, undecane, tridecane (Cetiol® Ultimate), dodecane, propylene glycol dipelargonate, diisopropyl sebacate, cetearyl isononanoate (Cetiol® SN), isononyl isononanoate, isocetyl stearoyl stearate, dipentaerithrityl hexacaprylate/hexacaprate, isodecyl neopentanoate, PEG-6 caprylic/ capric glycerides (Cetiol® 767), caprylic/capric triglyceride (Myritol® 312, Myritol® 318), ethylhexyl stearate, ethylhexylcocoate, ethylhexyl stearate (Cetiol® 868), dipropylheptyl carbonate (Cetiol® 4 All), hexyl laurate (Cetiol® A), dicaprylyl carbonate, PEG-7 glyceryl cocoate (Cetiol® HE), polyglyceryl-3 diisostearate (Lameform® TGI), lauryl alcohol, methyl canolate (Cetiol® MC), hexyldecyllaurate and hexyldecanol (Cetiol® PGL), hexyldecyl stearate (Eutanol® G 16S), PPG-15 Stearylether (CETIOL® E), ethylhexyl palmitat (CEGESOFT® C24), pentaerythrityl tetraethylhexanoate (Cetiol® PEEH4), hexyldecyllaurate and hexyldecanol (Cetiol® PGL), caprylyl caprylate/ caprate (Cetiol® RLF).

Further suitable oil components which can be used as emollients and incorporated in the beads are natural products selected from the group consisting of Elaeis guiineensis oil (Cegesoft® GPO), Passiflora incarnata seed oil (Cegesoft® PFO), olive oil, olus oil (Cegesoft® PS6), Butyrospermum parkii butter (Cetiol® SB 45), ethylhexylcocoat (and) Cocos Nucifera Öl (CETIOL® COCO), Shorea stenoptera seed butter (Cegesoft® SH), almond oil, avocado oil, borage oil, canola oil, castor oil, chamomile, coconut oil, corn oil, cottonseed oil, jojoba oil, evening primrose oil, papaya oil, palm oil, hazelnut oil, peanut oil, walnut oil, safflower oil, sesame oil, soybean oil, sunflower oil, sweet almond, a rice bran/wheat germ oil, rosehip oil, Ricinus communis oil, lanolin; hydrogenated vegetable oil, Candelilla cera, Euphorbia vegetable oil (Cegesoft® VP), sterols and derivatives.

In addition silicones and silicone derivatives such as polydimethylsiloxanes, methicone, dimethicone, cyclomethicone, caprylyl methicone, dimethicone copolyol, undecylcrylene dimethicone, dimethiconol, trimethicone, organosiloxanes are used as oil components in these loaded beads.

More preferably the emollients are selected from the group consisting of dibutyl adipate, caprylic/capric triglyceride, coco-caprylate/caprate, caprylyl caprylate, capric glycerides, coco-glycerides, C12-15 alkyl benzoate, propylheptyl caprylate, dicaprylyl carbonate, dicaprylyl ether, isohexadecane, isopropyl isostearate, octyldodecanol, volatile linear C8 to C16 alkanes, C10 to C15 alkanes, C11-C13 alkanes, C13-15 alkanes, C15-19 alkanes, isononyl isononanoate, PEG-6 Caprylic/ Capric Glycerides, Ethylhexyl Stearate, Dipropylheptyl Carbonate, Hexyl Laurate, Dicaprylyl Carbonate, Oleyl Erucate, PEG-7 Glyceryl Cocoate, Lauryl Alcohol, Methyl Canolate, Myristyl Myristate, Hexyldecyllaurate. Hexyldecanol, Cetearyl Isononanoate, Hexyldecyl Stearate, Polyglyceryl-3 Diisostearate, natural essential oils, mineral oil, parafinum liquidum, isododecane, undecane, dodecane, tridecane.

Most preferably the emollients are selected from the group consisting of dibutyl adipate, caprylic/capric triglyceride, coco-caprylate/caprate, caprylyl caprylate, capric glycerides, coco-glycerides, C12-15 alkyl benzoate, propylheptyl caprylate, dicaprylyl carbonate, dicaprylyl ether, isohexadecane, isopropyl isostearate, octyldodecanol, volatile linear C8 to C16 alkanes, isododecane, undecane, dodecane, tridecane, isononyl isononanoate, PEG-6 Caprylic/ Capric Glycerides, Ethylhexyl Stearate, Dipropylheptyl Carbonate, Hexyl Laurate, Dicaprylyl Carbonate, Oleyl Erucate, PEG-7 Glyceryl Cocoate, Lauryl Alcohol, Methyl Canolate, Myristyl Myristate, Hexyldecyllaurate. Hexyldecanol, Cetearyl Isononanoate, Hexyldecyl Stearate, Polyglyceryl-3 Diisostearate.

In a specific embodiment of the invention the oily ingredients are emollients selected from the group consisting of undecane, dodecane, tridecane, dibutyl adipate, dicaprylyl carbonate, dicaprylyl ether, propyl heptyl caprylate or mixtures thereof. These are preferred emollients which are often used as a substitute for silicones and silicone derivatives as there is an increasing demand to avoid the latter in cosmetic and personal care compositions for ecological reasons.

Preferably the oily ingredients have a melting point below 80°C, preferably the melting point is below 50°C and more preferably the melting point is below 30°C, most preferably the oily ingredients are liquid at room temperature (20 to 25 °C).

Preferably the oily ingredients have a boiling point above 100°C at 1 atmosphere of pressure (sea level), preferably the boiling point is above 130°C, more preferably the boiling point is above 150°C and most preferably the boiling point is above 180°C at 1 atmosphere of pressure (sea level). The higher the boiling point of the incorporated ingredient is, the lower the shrinkage during storage of the beads is.

**Cosmetic compositions**

Another embodiment of the invention is the use of the colored beads as peeling agent or for application of cosmetic ingredients, especially emollients on the skin.

The beads of the present invention can be incorporated into aqueous, semi-aqueous or nonaqueous cosmetic compositions. Cosmetic compositions shall mean any composition which is applied in contact with the various external parts of the human body (epidermis, hair system, nails, lips) or with the teeth and the mucous membranes of the oral cavity with the intend to cleaning them, perfuming them, changing their appearance and/or correcting body odours and/or protecting them or keeping them in good condition.

The cosmetic compositions according to the invention can for example be in the form of a hair shampoos, hair lotions, foam baths, shower baths, creams, gels, lotions, alcoholic and aqueous/alcoholic solutions, foams, emulsions, wax/fat masses, stick preparations, powders or ointments.

The cosmetic compositions comprise the spherical beads according to the invention in an amount of 0.1 to 50 weight-% based on the total weight of the composition, preferably in an amount of 1 to 30, especially in an amount of 2 to 10 weight-% based on the total weight of the composition.

Depending on their intended application, the cosmetic formulations contain a number of other auxiliaries and additives such as, for example, surface-active substances (surfactants, emulsifiers), other oil components, pearlizing waxes, consistency factors, thickeners, superfatting agents, stabilizers, polymers, fats, waxes, lecithins, phospholipids, biogenic agents, UV protection factors, antioxidants, deodorizers, antiperspirants, antidandruff agents, film formers, swelling agents, insect repellents, self-tanning agents, tyrosinase inhibitors (depigmenting agents), hydrotropes, solubilizers, preservatives, perfume oils, dyes and the like which are listed in the following.

The quantities in which the particular additives are used are determined by the intended application.

**Process for Manufacturing Cellulose Beads**

For the preparation of cellulose beads basically a solution of dissolved cellulose is prepared which could then be utilized to form cellulose beads by different techniques such as dropping, spraying, emulsification by $\mu$-fluidics or $\mu$-jet reactor.

The preferred manufacturing method is a three-step process comprising: 1. the dissolution, 2. precipitation and 3. loading, optionally a drying step can be added.
The respective dye can either be added to the dissolved cellulose solution (useful for dyes and pigments) in step 1 before precipitation or the wet precipitated cellulose beads can be soaked in a solution comprising the dye following step 3.

Manufacturing cellulose beads via **dropping** comprises the following steps:

1. Dissolution: A solution of cellulose at a concentration of 0.5 to 20 %, preferably 1 to 15 % by weight in an organic electrolyte solution comprising an organic solvent (52 - 88 by weight %; e.g. acetone, butanone, acetonitrile or acrylonitrile) and a quaternary ammonium salt (8 - 35 by weight %; e.g. tetrabutylammonium chloride, methyl tributylammonium chloride, tetrabutylammonium acetate, methyl tributylammonium acetate, tetrapropylammonium acetate, tetrabutylammonium acrylate, methyl tributylammonium acrylate) is prepared according to the procedures of European application WO2019/081246. The dyes and pigments can be added by dissolution or dispersion in the cellulose solution. Optionally other active ingredients (liquids, solids, polymers, enzymes) can be introduced during dissolution.

2. Droplet Formation/Precipitation: The solution is then dropped into a precipitation bath of a protic solvent or into a precipitation bath of mixtures of solvents, where one of the solvents is protic, preferably selected from the group

consisting of $H_2O$, ethanol, methanol, propanol, isopropanol, butanol, glycerin, solutions of organic or inorganic salts, buffer solution, acetonitrile/ethanol 90:10; dichloromethane/ethanol 90:10; water/acetone 50:50 and mixtures thereof. More preferably the solvent of the precipitation bath is selected from the group consisting of ethanol or propanol, most preferably ethanol.

The droplet formation occurs in the gas phase 0.1 to 100 cm, preferably 0.5 to 30 cm, above the surface of the precipitation bath (air, nitrogen, argon, or vapor of the precipitation bath) by passing the solution through an open end canula, tube, pipette, nozzle or any other system (i.e. vibrating nozzle) to form droplets. Upon contact with the liquid phase of the precipitation bath, the droplets solidify instantaneously and remain in droplet form as individual separate particles. The resulting beads may directly sink to the bottom of the precipitation bath (example: ethanol, methanol) or stay at the surface (example: dichloromethane/ethanol) or stay at the surface of the precipitation bath at first and sink to the bottom when fully equilibrated with precipitation medium (example: water), depending on the density of the precipitation medium.

3. Isolation/Filtration and Loading: The resulting soaked (with precipitation medium) beads are isolated by filtration and may be re-dispersed in a fresh precipitation bath. This washing procedure can be repeated several times until no residuals of the organic electrolyte solution can be detected by elemental analysis. The isolated beads soaked with precipitation medium can be either dried (air, vacuum, heat) or dispersed in an active ingredient (oils, salt solutions, polymer solutions, enzyme solutions) or solutions thereof. The dyes and pigments could be added at this loading step. Upon complete equilibration the beads may be isolated by filtration.

Manufacturing cellulose beads via **spraying** comprises the following steps:

1. Dissolution: A solution of cellulose at a concentration of 1.0 to 10 %, preferably 2 to 5 % by weight in an organic electrolyte solution comprising an organic solvent (52 - 88 %; e.g. acetone, butanone, acetonitrile or acrylonitrile) and a quaternary ammonium salt (8 - 35 %; e.g. tetrabutylammonium chloride, methyl tributylammonium chloride, tetrabutylammonium acetate, methyl tributylammonium acetate, tetrapropylammonium acetate, tetrabutylammonium acrylate, methyl tributylammonium acrylate) is prepared according to the procedures of WO2019/081246. Optionally active ingredients and or dyes (e.g. liquids, solids, polymers, enzymes) can be introduced to the solution by either dissolution or dispersion in the cellulose solution.

2. Precipitation: The solution is then sprayed into a precipitation bath of a protic solvent or into a precipitation bath of mixtures of solvents, where one of the solvents is protic, preferably selected from the group consisting of H2O, ethanol, methanol, propanol, butanol, glycerin, solutions of organic or inorganic salts, buffer solution, acetonitrile/ethanol 90:10; dichloromethane/ethanol 90:10; water/acetone 50:50 and mixtures thereof.

The droplet formation occurs in the gas phase 0.1 to 100 cm, preferably 0.5 to 30 cm, above the surface of the precipitation bath (air, nitrogen, argon, or vapor of the precipitation bath) by passing the cellulose solution through a nozzle with high pressure. Upon contact with the liquid phase of the precipitation bath, the droplets solidify instantaneously and remain in droplet form as individual separate particles. The resulting beads directly sink to the bottom of the precipitation bath (ethanol, methanol, etc.).

3. Isolation/Filtration: The resulting soaked (with precipitation medium) beads are isolated by filtration and may be redispersed in a fresh precipitation bath. This washing procedure can be repeated several times until no residuals of the organic electrolyte solution can be detected by elemental analysis. The isolated beads soaked with precipitation medium can be either dried (air, vacuum, heat) or dispersed in an active ingredient (dyes, oils, salt solutions, polymer solutions, enzyme solutions) or solutions thereof. Upon complete equilibration the beads may be isolated be filtration. The bead size was measured by laser diffraction and optical microscopy.

Manufacturing cellulose beads via **emulsification** comprising the following steps:

1. Dissolution: A solution of cellulose at a concentration of 1.0 to 10 %, preferably 2 to 5 % by weight in an organic electrolyte solution comprising an organic solvent (52 - 88 %; e.g. acetone, butanone, acetonitrile or acrylonitrile) and a quaternary ammonium salt (8 - 35 %; e.g. tetrabutylammonium chloride, methyl tributylammonium chloride, tetrabutylammonium acetate, methyl tributylammonium acetate, tetrapropylammonium acetate, tetrabutylammonium acrylate, methyl tributylammonium acrylate) is prepared according to the procedures of WO2019/081246. Optionally active ingredients and/or dyes (e.g. liquids, solids, polymers, enzymes) can be introduced to the solution by either dissolution or dispersion in the cellulose solution.

2. Emulsification: The cellulose solution is emulsified in an oil phase consisting of a predominantly hydrophobic oil such as for example an emollient or plant oil and a surfactant in a ratio of cellulose solution to oil phase of 1:1 to 1:10, preferably 1:1 to 1:5 more preferably 1:2 to 1:3 using an Ultra Turrax@ at 10000 rpm for 30 s. Alternatively the emulsification can be conducted with a μ-jet reactor, via μ-Fluidics or any other instrument available for emulsification.

3. Precipitation: The emulsion is then dropwise added to a precipitation bath of a protic solvent or into a precipitation bath of mixtures of solvents, where one of the solvents is protic, preferably selected from the group consisting of H2O, ethanol, methanol, propanol, butanol, glycerin, solutions of organic or inorganic salts, buffer solution, acetonitrile/ethanol 90:10; dichloromethane/ethanol 90:10; water/acetone 50:50 and mixtures thereof.

Upon contact with the liquid phase of the precipitation bath, the droplets solidify instantaneously and remain in droplet form as individual separate particles.

4. Isolation/Filtration: The resulting soaked (with precipitation medium) beads are isolated by centrifugation (3500 rpm, 10 min) and may be redispersed in a fresh precipitation bath. This washing procedure can be repeated several times until no residuals of the organic electrolyte solution can be detected. The isolated beads soaked with precipitation medium can be either dried (air, vacuum, heat) or dispersed in an active ingredient (dyes, oils, salt solutions, polymer solutions, enzyme solutions) or solutions thereof. Upon complete equilibration the beads may be isolated be filtration. The bead size was measured by laser diffraction.

## Specific examples for of preparing cellulose beads

Raw materials

Cellulose:

In general, all types of cellulose materials with a predominant amount of cellulose can be used for this process. The process parameters for dissolution (time, viscosity) may vary for cellulose materials of different molecular weight. The cellulose used in the current examples was microcrystalline cellulose for short referred to as MCC - microcrystalline cellulose (Source: Sigma-Aldrich, characteristics: degree of crystallinity according to solide state 13C-NMR: 57 %, see below.)

Following quaternary ammonium compounds (tetra-butyl-ammonium - TBA-) have been used:

Tetra-butyl-ammonium-acetate, (TBA-Acetate)
Tetra-butyl-ammonium-acrylate, (TBA-Acrylate, TBAAc)
Tri-butyl-methyl-ammonium-acetate, (TBMA-Acetate)
Allyl-tri-butyl-ammonium-acetate, (ATBA-Acetate)

TBA-Acetate and TBA-Acrylate have been prepared via anion exchange from TBA-chloride obtained from Sigma Aldrich. TBMA-Acetate has been prepared via anion exchange from TBMA-chloride obtained from Sigma Aldrich.
ATBA-Acetate war prepared according to the following procedure:
First, quaternary ammonium bromide was prepared from tributyl-amine and allylbromide. Thereafter the bromide was replaced by acetate via anion exchange according to the following procedure: A column, loaded with 10 g of acetate ion exchange resin was loaded with a solution of 2.0 g of allyltripropylammonium bromide dissolved in 30 mL of methanol. The solution was passed over the resin with a flow rate of 1 ml/min and the resin was washed with additional 30 ml of MeOH afterwards. After removing the solvents, the crude product was dried in vacuo at 40 °C to quantitatively yield allyl-tri-butyl-ammonium-acetate.

Dyes and pigments:

Puricolor®-types (BTC Europe GmbH, Monheim am Rhein, Germany)

Puricolor Yellow FYE 13
Puricolor Red ARE 33 FDA
Puricolor Green U3 FDA
Puricolor Red ARE 33 FDA

Benzopurpurin 4B (Direct Red 2, CAS-992-59-6; TCI Deutschland GmbH, Eschborn, Germany)
Brilliant Blue R (CAS-6104-59-2; BASF SE, Ludwigshafen, Germany)

Sicopal Orange L2430 (Sicopal®-products, BTC Europe GmbH, Monheim am Rhein, Germany)

Emollients (Oily Ingredients):

Cetiol®-types by BASF Personal Care ad Nutrition GmbH, Germany:

Cetiol®B - Dibutyl Adipate
Cetiol® CC - Dicaprylyl Carbonate
Cetiol® OE - Dicaprylyl Ether
Cetiol® Ultimate - Undecane, Tridecane

Dissolution of cellulose

**Examples for the dissolution of cellulose (summarized in Table 1):**

Procedure for the dissolution of cellulose in TBAAc / MeCN (Ex. 1.1 - Ex. 1.5 and Ex. 1.9) Cellulose (29.5 g) was added to a solution of tetrabutylammonium acetate (95.3 g) in acetonitrile (160 g) and stirred for 30 min at ambient temperature. The temperature was increased to 65°C and the solvent was removed under reduced pressure. Acetonitrile (300 g) was dosed to the vessel over a period of 9 h at a temperature of 65°C to obtain a clear, colorless solution. The obtained solution (424.8 g) with a composition of cellulose / TBAAc / acetonitrile (6.9 / 22.4 / 70.6 wt%) was further diluted with acetonitrile and TBAAc to yield the desired composition.

Procedure for the dissolution of cellulose in TBAAc / MeCN (Ex. 1.6 - Ex. 1.8 and Ex 1.10 - Ex. 1.18) Cellulose (3.0 - 13.0 wt%) was added to a solution of tetrabutylammonium acetate (8.4 - 36.5 wt%) in acetonitrile (100 - 300 mL) and stirred for 30 min at ambient temperature. The temperature was increased to 65°C and the solvent was removed under reduced pressure. Acetonitrile (50.1 - 88.6 wt%) was dosed to the vessel over a period of 9 h at a temperature of 65°C to obtain a clear, colorless solution.

Procedure for the dissolution of cellulose in TBAC/acetone (Ex. 1.19 and Ex. 1.21) 70.0 g of TBAC, 7.0 g of MCC and 35.0 g of acetone were added to a 250 mL four-necked flask. The mixture was stirred at 50 °C for 10 min. The volatiles were removed at 125 °C external temperature. After cooling to 85 °C, the viscous melt was stirred for 15 h. The desired amount of acetone was added to the clear, orange melt in 10 min, which was cooled down to 65 °C. The final solution was stirred at 65 °C for 30 min.

Table 1: Examples for cellulose cellulose solutions for the preparation of beads.

| Example | QUAT | Solvent | Cellulose (wt%) | QUAT (wt%) | Solvent (wt%) |
|---|---|---|---|---|---|
| **Ex. 1.1** | TBAAc | MeCN | 0.5 | 5.0 | 94.5 |
| **Ex. 1.2** | TBAAc | MeCN | 0.5 | 14.6 | 84.9 |
| **Ex. 1.3** | TBAAc | MeCN | 1.0 | 14.4 | 84.6 |
| **Ex. 1.4** | TBAAc | MeCN | 2.5 | 20.6 | 76.9 |
| **Ex. 1.5** | TBAAc | MeCN | 2.5 | 28.4 | 69.1 |
| **Ex. 1.6** | TBAAc | MeCN | 3.0 | 8.4 | 88.6 |
| **Ex. 1.7** | TBAAc | MeCN | 3.0 | 14.2 | 82.8 |
| **Ex. 1.8** | TBAAc | MeCN | 5.0 | 13.9 | 81.1 |
| **Ex. 1.9** | TBAAc | MeCN | 5.0 | 14.3 | 80.7 |
| **Ex. 1.10** | TBAAc | MeCN | 5.0 | 17.5 | 77.5 |
| **Ex. 1.11** | TBAAc | MeCN | 5.0 | 27.4 | 67.6 |
| **Ex. 1.12** | TBAAc | MeCN | 6.0 | 26.3 | 67.7 |
| **Ex. 1.13** | TBAAc | MeCN | 6.25 | 28.3 | 65.4 |
| **Ex. 1.14** | TBAAc | MeCN | 6.9 | 22.4 | 70.6 |
| **Ex. 1.15** | TBAAc | MeCN | 10.0 | 27.8 | 62.2 |
| **Ex. 1.16** | TBAAc | MeCN | 10.0 | 33.4 | 56.6 |

(continued)

| Example | QUAT | Solvent | Cellulose (wt%) | QUAT (wt%) | Solvent (wt%) |
|---|---|---|---|---|---|
| Ex. 1.17 | TBAAc | MeCN | 10.9 | 36.5 | 52.6 |
| Ex. 1.18 | TBAAc | MeCN | 13.0 | 36.1 | 50.9 |
| Ex. 1.19 | TBAC | Acetone | 3.2 | 32.3 | 64.5 |
| Ex. 1.20 | TBAC | Acetone | 4.6 | 44.6 | 50.8 |
| Ex. 1.21 | TBAC | Acetone | 4.0 | 30.0 | 64.0 |

**Examples for the preparation of cellulose beads (summarized in Table 2)**

Equipment: 3 l beaker with PTFE half-moon-blade stirrer and dropping funnel

The beaker was charged with 500 g of ethanol. With a rate of 1 drop per second, 10.0 g of cellulose solution (Ex. 1.1 - Ex. 1.20) were dropped from a height of 6 cm into the stirred ethanol. The stirrer was stopped after completion of the feed. The obtained cellulose beads sink to the bottom of the breaker immediately upon addition to the ethanol. The cellulose beads were isolated by filtration. The cellulose beads were suspended in 2000 ml fresh ethanol for 1 h and isolated by filtration. This washing step including suspention and filtration was repeated one more time. The resulting isolated beads were dried in vacuum (40°C, 2 h, 100 mbar).

In order to verify that the washing procedure was sufficiently repeated until no residuals of the organic electrolyte solution could be detected an elemental analysis of the cellulose beads of example 2.3 was conducted, whereby the microcrystalline cellulose (MCC) was subjected to the same washing steps as the beads. The results of the elemental analysis for the MCC were: Carbon 43.5 g/100 g, Oxygen 50 g/100 g, Nitrogen < 0.5 g /100 g, Hydrogen 6.7 g /100 g, the respective values of the cellulose beads resulted in Carbon 43.4 g/100 g, Oxygen 50 g/100 g, Nitrogen < 0.5 g /100 g. Hence no residuals of the solvents could be detected.

Table 2: Examples of pure cellulose beads. Manufactured for $\mu$-CT und NMR measurements:

| Example | Solution Number | Cellulose concentratio n of solution (wt.%) | Cellulose content in beads (wt.%) | Water content (wt.%) | Bead size [mm] |
|---|---|---|---|---|---|
| Ex. 2.1 | Ex. 1.17 | 10.9 | > 99.5 | < 0.5 | 1.90 |
| Ex. 2.2 | Ex. 1.16 | 10.0 | > 99.5 | < 0.5 | 1.05 |
| Ex. 2.3 | Ex. 1.9 | 5.0 | > 99.5 | < 0.5 | 0.84 |

**Examples for the preparation of cellulose beads containing oily ingredients - inventive examples (summarized in Table 3)**

Beads produced from TBAAc/MeCN loaded with Cetiol types (Ex. 3.1 - Ex. 3.4 and Ex. 3.12 - Ex. 3.17): Equipment: 3 l beaker with PTFE half-moon-blade stirrer and three diaphragm pump SIMDOS 02 from KNF.

The beaker was charged with 2000 ml of ethanol. With a rate of 3 mL/min, 193.6 g of cellulose solution in **TBAAc/MeCN** (Ex. 1.1 - Ex. 1.18) were dropped from a height of 0.5 cm through a canula with a diameter of 0.95 mm into the stirred ethanol. The stirrer was stopped after completion of the feed. The obtained cellulose beads sink to the bottom of the breaker immediately upon addition to the ethanol. 1 h after completion of the feed, the cellulose beads were isolated by filtration. The cellulose beads were suspended in 2000 ml fresh ethanol for 1 h and isolated by filtration. This washing step including suspension and filtration was repeated one more time.

69.1 g of isolated beads were added to a mixture of 251.3 g of Cetiol type (see table 3) and 249.8 g of ethanol in a 1 L glass beaker. After 1 h the cellulose beads were isolated by filtration and added 502.6 g of fresh Cetiol. This step was repeated with 507.1 g of Cetiol without EtOH. The open vial with Cetiol and cellulose beads were stored for 2 days in a vacuum cabinet at 50 mbar and ambient temperature. The cellulose beads were isolated by filtration. The weight of the isolated beads was 62.4 g. The beads were stored in a closed container at ambient conditions under air atmosphere.

Beads produced from **TBAC/acetone** loaded with Cetiol types (Ex. 3.6 - Ex. 3.9):

Equipment: 3 L beaker with PTFE half-moon-blade stirrer and three diaphragm pump SIMDOS 02 from KNF.

The beaker was charged with 2000 mL of ethanol. With a rate of 3 mL/min, 193.6 g of cellulose solution in TBAC/acetone (Ex. 1.19) were dropped from a height of 0.5 cm through a canula with a diameter of 0.95 mm into the stirred ethanol. The stirrer was stopped after completion of the feed. The obtained cellulose beads sink to the bottom of the breaker immediately upon addition to the ethanol. 1 h after completion of the feed, the cellulose beads were isolated by filtration. The cellulose beads were suspended in 2000 mL fresh ethanol for 1 h. This washing step was repeated. 69.1 g of isolated beads were added to a mixture of 251.3 g of Cetiol (see table 3) and 249.8 g of ethanol in a 1 L glass beaker. After 1 h the cellulose beads were isolated by filtration and added 502.6 g of fresh Cetiol. This step was repeated with 507.1 g of Cetiol. The open vial with Cetiol and cellulose beads were stored for 2 days in a vacuum cabinet at 50 mbar and ambient temperature. The cellulose beads were isolated by filtration. The weight of the isolated beads was 62.4 g. The beads were stored in a closed container at ambient conditions under air atmosphere.

**Beads loaded with a mint mixture (Ex. 3.10):**

Equipment: 3 L beaker with PTFE half-moon-blade stirrer and dropping funnel

The beaker was charged with 1000 g of ethanol. With a rate of 1 mL/min, 100.0 g of cellulose solution in TBAAc/MeCN (Ex. 1.4) were dropped from a height of 6 cm through a canula with a diameter of 0.95 mm into the stirred ethanol. The stirrer was stopped after completion of the feed. The obtained cellulose beads sink to the bottom of the breaker immediately upon addition to the ethanol. The cellulose capsules were isolated by filtration. The weight of the isolated wet beads was 27.1 g and the solid content was 11.9 wt.%. The capsule diameter d50 was 2.4 mm. 10 g of the wet capsules were dispersed in 50 g of a mixture of L-Isopulegol (23.5 g), L-Menthylacetat (32.0 g), L-menthon (365 g) and L-Menthol (582.5 g) for 24 h. 10.7 g of wet beads with a solid content of 9.73 g with a diameter of d50 of 2.4 mm (determined by light microscopy) could be isolated by filtration.

**Beads loaded with a parfume mixture (Ex. 3.11):**

Equipment: 50 mL vial and dropping funnel

The vial was charged with 20 g of ethanol. With a rate of 1 mL/min, 1.0 g of cellulose solution in TBAAc/MeCN (Ex. 1.17) were dropped from a height of 6 cm through a canula with a diameter of 0.95 mm into the stirred ethanol. The stirrer was stopped after completion of the feed. The obtained cellulose beads sink to the bottom of the vial immediately upon addition to the ethanol. The cellulose beads were isolated by filtration. The wet beads were dispersed in 10 g of a parfume mixture (Perfurme Oil COWBOY Super 719F, BASF) for 24 h and isolated by filtration.

Table 3: Examples for beads containing oily ingredients.

| Examples | Cellulose solution | Bead size [mm] | Active ingredient | Theoretical active concentration [%] | Experimental active concentration [%] |
|----------|--------------------|----------------|-------------------|--------------------------------------|---------------------------------------|
| Ex. 3.1 | Ex. 1.4 | 2.0 | CetiolOE | 97.5 | 95.8 |
| Ex. 3.2 | Ex. 1.4 | 2.0 | Cetiol B | 97.5 | 96.5 |
| Ex. 3.3 | Ex. 1.4 | 2.0 | Cetiol CC | 97.5 | 96.0 |
| Ex. 3.4 | Ex. 1.4 | 2.0 | Cetiol Ultimate | 97.5 | 95.5 |
| Ex. 3.5 | Ex. 1.4 | 0.5 | Cetiol OE | 97.5 | - |
| Ex. 3.6 | Ex. 1.20 | 2.0 | Cetiol OE | 95.4 | 92.3 |
| Ex. 3.7 | Ex. 1.20 | 2.0 | Cetiol Ultimate | 95.4 | 91.7 |
| Ex. 3.8 | Ex. 1.20 | 2.0 | Cetiol CC | 95.4 | 93.5 |
| Ex. 3.9 | Ex. 1.20 | 2.0 | Cetiol B | 95.4 | 94.4 |
| Ex. 3.10 | Ex. 1.4 | 2.4 | Mint mix | 75.0 | - |

(continued)

| Examples | Cellulose solution | Bead size [mm] | Active ingredient | Theoretical active concentration [%] | Experimental active concentration [%] |
|---|---|---|---|---|---|
| Ex. 3.11 | Ex. 1.17 | - | Parfume mix | 89.1 | - |
| Ex. 3.12 | Ex. 1.5 | 1.1 | Cetiol OE | 97.5 | 97.2 |
| Ex. 3.13 | Ex. 1.13 | 1.4 | Cetiol OE | 93.75 | 90.5 |
| Ex. 3.14 | Ex. 1.13 | 1.9 | Cetiol OE | 93.75 | 89.7 |
| Ex. 3.15 | Ex. 1.16 | 2.3 | Cetiol OE | 90.0 | 81.7 |
| Ex. 3.16 | Ex. 1.16 | 2.1 | Cetiol OE | 90.0 | 84.0 |
| Ex. 3.17 | Ex. 1.11 | 1.7 | Cetiol OE | 95.0 | 92.2 |

The loaded beads were subjected to pressure profile measurements (results see table 4).

Table 4: Pressure profile measurements

| Beads from example | Active content (CetiolOE) [wt.%] | Cellulose content [wt.%] | Cellulose in solution [wt.%] | Average Diameter D (4,3) [μm] | Average Force 20% Way [N] | Average Force 50% Way [N] | Average Force 80% Way [N] |
|---|---|---|---|---|---|---|---|
| Comparative material "Vivapor CS 800 S" | 0.0 | 100 | | 932 | 32.5 | 36.6 | 73.4 |
| Comparative Ex. 2.2 | 0.0 | 100 | 10,00 | 1053 | 42.8 | 100.0 | |
| Ex. 3.12 | 97.2 | 2,8 | 2,50 | 1067 | 0.2 | 1.0 | 3.5 |
| Ex. 3.13 | 90.5 | 9,5 | 6,25 | 1412 | 2.9 | 9.3 | 22.3 |
| Ex. 3.14 | 89.7 | 10,3 | 6,25 | 1891 | 5.2 | 18.0 | 37.4 |
| Ex. 3.15 | 81.7 | 18,3 | 10,00 | 2289 | 19.9 | 57.6 | 137.1 |
| Ex. 3.16 | 84.0 | 16,0 | 10,00 | 2070 | 14.8 | 48.9 | 120.4 |
| Ex. 3.17 | 92.2 | 7,8 | 5,00 | 1701 | 2.4 | 9.8 | 21.7 |

**Examples for the preparation of cellulose beads containing dyes**

See tables 5 and 6 for an overview of beads containing dyes and cellulose

**Ex. 4.1a for beads containing a yellow dye**

Equipment: 1 L beaker with PTFE half-moon-blade stirrer and dropping funnel

The beaker was charged with 500 g of ethanol. Yellow FYE 13 (0.0122 g) was dissolved in 6.0 g of cellulose solution in TBAAc/MeCN (10.0 wt.% MCC; weight ratio Cellulose: Yellow FYE 13 = 98 : 2) and the solution was dropped from a height of 6 cm to the stirred ethanol. The stirrer was stopped after completion of the feed. The obtained cellulose beads sink to the bottom of the breaker immediately upon addition to the ethanol. The beads were isolated by filtration. After drying the diameter of the beads was 1.9 as determined by light microscopy.

**Ex. 4.1b for beads containing a yellow dye**

Equipment: 1 L beaker with PTFE half-moon-blade stirrer and dropping funnel

The beaker was charged with 500 g of ethanol. 6.0 g of cellulose solution in TBAAc/MeCN (10.0 wt.% MCC) was dropped from a height of 6 cm to the stirred ethanol. The stirrer was stopped after completion of the feed. The obtained cellulose beads sink to the bottom of the breaker immediately upon addition to the ethanol. The cellulose beads were isolated by filtration and the beads were washed in ethanol. The diameter of the wet capsules was d50 = 2.0 mm as determined by microscopy. 1.01 g of wet cellulose beads were dispersed in an aqueous solution of Puricolor Yellow FYE 13 (25 wt.%) over night and isolated by filtration. The particles were dried and 0.298 g of yellow cellulose beads were obtained.

**Ex. 4.2 for beads containing a red dye**

Equipment: 1 L beaker with PTFE half-moon-blade stirrer and dropping funnel

The beaker was charged with 500 g of ethanol. Puricolor Red ARE 33 FDA (0.0069 g) was dissolved in 6.0 g of cellulose solution in TBAC/acetone (4.0 wt.% MCC; weight ratio Cellulose: Puricolor Red ARE 33 FDA = 98:2) and the solution was dropped from a height of 6 cm to the stirred ethanol. The stirrer was stopped after completion of the feed. The obtained cellulose beads sink to the bottom of the breaker immediately upon addition to the ethanol. The cellulose capsules were isolated by filtration. After drying the diameter of the capsules was 1.9 mm as determined by light microscopy.

**Ex. 4.3 for beads containing a green dye**

Equipment: 1 L beaker with PTFE half-moon-blade stirrer and dropping funnel

The beaker was charged with 500 g of ethanol. Puricolor Green U3 FDA (0.0069 g) was dissolved in 6.0 g of cellulose solution in TBAC/acetone (4.0 wt.% MCC, weight ratio Cellulose: Puricolor Green U3 FDA = 98:2) and the solution was dropped from a height of 6 cm to the stirred ethanol. The stirrer was stopped after completion of the feed. The obtained cellulose beads sink to the bottom of the breaker immediately upon addition to the ethanol. The cellulose capsules were isolated by filtration. After drying the diameter of the capsules was 1.9 mm as determined by light microscopy.

**Ex. 4.4 for beads containing a red dye**

Equipment: 10 ml vial and an Ultraturrax rotor-stator device.

1.0 ml of cellulose solution in TBAAc/MeCN (10.0 wt.% MCC) containing 0.4 mg of benzopurpurin 4B was emulsified in 4.0 ml of dicaprylyl ether (Cetiol® OE) containing 0.05 g of a surfactant mixture (comprising 39.7 wt% Arlacel P 135, 39.7 wt% Span 85, 10.3 wt% Span 80, 10.3 wt % Cremophor A6 based on the weigt of the surfactant mixture) using an Ultraturrax rotor-stator device (30s, 10 000 rpm). The obtained emulsion was then transferred into 100 ml ethanol to precipitate the cellulose droplets. The obtained dispersion was washed 2 times with 100 ml of ethanol. The final bead diameter was d50= 2.5 $\mu$m as determined by light scattering.

**Ex. 4.5 and 4.8 - beads containing a red dye**

Equipment: 50 mL vials and 3 mL pipet

The vial was charged with 30 g of ethanol. Direct Red 83.1 (0.0020 g) was dispersed in 3.85 g of cellulose solution in TBBAc/MeCN (10 wt.% MCC; weight ratio cellulose : Direct Red 83.1 = 99.6:0.4) and the solution was dropped from a height of 1 cm to the ethanol. The obtained cellulose beads sink to the bottom of the breaker immediately upon addition to the ethanol. The cellulose capsules were left in the ethanol for 30 min and isolated by filtration, The beads were suspended in 30 mL fresh ethanol for 30 min and isolated by filtration.

**Ex. 4.6 for beads containing a blue dye**

Equipment: 50 mL vial and 0.9 mm needle.

The vial was charged with 50 g of ethanol. Brilliant Blue R was dissolved in acetonitrile 0.11 wt.%. 1.00 g Brilliant Blue R solution (0.10 wt%) in 10 g of cellulose solution in TBBAC/MeCN (10 wt.% MCC; weight ratio Cellulose : Brilliant Blue R = 99.9:0.1) and the solution was dropped from a height of 1 cm to the ethanol. The obtained cellulose beads sink to the bottom of the breaker immediately upon addition to the ethanol. The cellulose beads were left in ethanol for 30 min and isolated by filtration. Two consecutive washing steps were conducted by suspending the beads in fresh ethanol (60 g / 86 g) for 30 min and isolating them by filtration. The particles were dried at 40°C in vacuum overnight. The diameter of the dry capsules was 1.1 mm as determined by microscopy.

**Ex. 4.7 and Ex. 4.11** - **beads containing an orange dye**

Equipment: 50 mL vials and 0.9 mm needle

The vial was charged with 50 g of ethanol. Sicopal Orange L2430 (0.0051 g) was dispersed in 10 g of cellulose solution in TBAAC/MeCN (10 wt.% MCC; weight ratio Cellulose : Sicopal Orange L2430 = 99.5:0.5) and the solution was dropped from a height of 1 cm to the ethanol. The obtained cellulose beads sink to the bottom of the breaker immediately upon addition to the ethanol. The cellulose beads were left in ethanol for 30 min and isolated by filtration. Two consecutive washing steps were conducted by suspending the beads in fresh ethanol (200 g / 200 g) for 30 min and isolating them by filtration. The particles were dried at 40°C in vacuum overnight.

**Ex. 4.9 - beads containing oil and a red dye**

Equipment: 50 mL vials and 0.9 mm needle

The vial was charged with 50 g of ethanol. Direct Red 83.1 (0.0051 g) was dispersed in 10 g of cellulose solution in TBAAC/MeCN (10 wt.% MCC; weight ratio Cellulose : Direct Red 83.1 = 99.5:0.5) and the solution was dropped from a height of 1 cm to the ethanol. The obtained cellulose beads sink to the bottom of the breaker immediately upon addition to the ethanol. The cellulose beads were left in ethanol for 30 min and isolated by filtration. Two consecutive washing steps were conducted by suspending the beads in fresh ethanol (200 g / 200 g) for 30 min and isolating them by filtration. The isolated beads were redispersed in a solution of Cetiol OE in ethanol (6.3%, 200 g) for 30 min and isolated by filtration. The particles were dried at 40°C in vacuum overnight.

**Ex. 4.10 - beads containing oil and a red dye**

Equipment: 50 mL vials and 0.9 mm needle

The vial was charged with 50 g of ethanol. Direct Red 83.1 (0.0051 g) was dispersed in 10 g of cellulose solution in TBAAC/MeCN (10 wt.% MCC; weight ratio Cellulose : Direct Red 83.1 = 99.5:0.5) and the solution was dropped from a height of 1 cm to the ethanol. The obtained cellulose beads sink to the bottom of the breaker immediately upon addition to the ethanol. The cellulose beads were left in ethanol for 30 min and isolated by filtration. Two consecutive washing steps were conducted by suspending the beads in fresh ethanol (200 g / 200 g) for 30 min and isolating them by filtration. The isolated beads were two times redispersed in Cetiol OE (200 g) for 30 min and isolated by filtration. The particles were dried at 40°C in vacuum overnight.

**Ex. 4.12 - beads containing oil and an orange dye**

Equipment: 50 mL vials and 0.9 mm needle

The vial was charged with 50 g of ethanol. Sicopal Orange L2430 (0.0051 g) was dispersed in 10 g of cellulose solution in TBAAC/MeCN (10 wt.% MCC; weight ratio Cellulose : Sicopal Orange L2430 = 99.5:0.5) and the solution was dropped from a height of 1 cm to the ethanol. The obtained cellulose beads sink to the bottom of the breaker immediately upon addition to the ethanol. The cellulose beads were left in ethanol for 30 min and isolated by filtration. Two consecutive washing steps were conducted by suspending the beads in fresh ethanol (200 g / 200 g) for 30 min and isolating them by filtration. The isolated beads were redispersed in a solution of Cetiol OE in ethanol (6.3%, 200 g) for 30 min and isolated by filtration. The particles were dried at 40°C in vacuum overnight.

**Ex. 4.13 - beads containing oil and an orange dye**

Equipment: 50 mL vials and 0.9 mm needle

The vial was charged with 50 g of ethanol. Sicopal Orange L2430 (0.0051 g) was dispersed in 10 g of cellulose solution in TBAAC/MeCN (10 wt.% MCC; weight ratio Cellulose : Sicopal Orange L2430 = 99.5:0.5) and the solution was dropped from a height of 1 cm to the ethanol. The obtained cellulose beads sink to the bottom of the breaker immediately upon addition to the ethanol. The cellulose beads were left in ethanol for 30 min and isolated by filtration. Two consecutive washing steps were conducted by suspending the beads in fresh ethanol (200 g / 200 g) for 30 min and isolating them by filtration. The isolated beads were two times redispersed in Cetiol OE (200 g) for 30 min and isolated by filtration. The particles were dried at 40°C in vacuum overnight.

**Ex. 4.14 for beads containing a blue dye**

Equipment: 50 mL vial and 0.9 mm needle.

The vial was charged with 50 g of ethanol. 10 g of cellulose solution in TBAC/MeCN (6 wt.% MCC) was dropped from a height of 1 cm to the ethanol. The obtained cellulose beads sink to the bottom of the breaker immediately upon addition to the ethanol. The cellulose beads were left in ethanol for 30 min and isolated by filtration. Two consecutive washing steps were conducted by suspending the beads in fresh ethanol (200 g / 200 g) for 30 min and isolating them by filtration. The beads were redispersed in a solution of Brilliant Blue R in ethanol for 30 min and isolated by filtration. The particles were dried at 40°C in vacuum overnight.

**Ex. 4.15 - beads containing oil and a blue dye**

Equipment: 50 mL vial and 0.9 mm needle.

The vial was charged with 50 g of ethanol. 10 g of cellulose solution in TBAC/MeCN (6 wt.% MCC) was dropped from a height of 1 cm to the ethanol. The obtained cellulose beads sink to the bottom of the breaker immediately upon addition to the ethanol. The cellulose beads were left in ethanol for 30 min and isolated by filtration. Two consecutive washing steps were conducted by suspending the beads in fresh ethanol (200 g / 200 g) for 30 min and isolating them by filtration. The beads were redispersed in a solution of Brilliant Blue R in ethanol for 30 min and isolated by filtration. The isolated beads were redispersed in a solution of Cetiol OE in ethanol (6.3%, 200 g) for 30 min and isolated by filtration. The particles were dried at 40°C in vacuum overnight.

**Ex. 4.16 - beads containing oil and a blue dye**

Equipment: 50 mL vial and 0.9 mm needle.

The vial was charged with 50 g of ethanol. 10 g of cellulose solution in TBAC/MeCN (6 wt.% MCC) was dropped from a height of 1 cm to the ethanol. The obtained cellulose beads sink to the bottom of the breaker immediately upon addition to the ethanol. The cellulose beads were left in ethanol for 30 min and isolated by filtration. Two consecutive washing steps were conducted by suspending the beads in fresh ethanol (200 g / 200 g) for 30 min and isolating them by filtration. The beads were redispersed in a solution of Brilliant Blue R in ethanol for 30 min and isolated by filtration. The isolated beads were redispersed in a solution of Cetiol OE in ethanol (6.3%, 200 g) for 30 min and isolated by filtration.. The isolated beads were two times redispersed in Cetiol OE (200 g) for 30 min and isolated by filtration. The particles were dried at 40°C in vacuum overnight.

Table 5: Overview of beads containing dyes and cellulose

| Example | Cellulose solution | Bead size (mm) | Dye | Cellulose content (wt. %) | Dye content (wt.%) | Experimental oil content (wt.% total bead) |
|---|---|---|---|---|---|---|
| Ex. 4.1a and Ex. 4.1b | Ex. 1.16 | 2.0 | Yellow FYE 13 | 98 | 2 | 0 |

(continued)

| Example | Cellulose solution | Bead size (mm) | Dye | Cellulose content (wt.%) | Dye content (wt.%) | Experimental oil content (wt.% total bead) |
|---|---|---|---|---|---|---|
| Ex. 4.2 | Ex. 1.21 | 1.9 | Puricolor Red ARE 33 FDA | 98 | 2 | 0 |
| Ex. 4.3 | Ex. 1.21 | 1.9 | Puricolor Green U3 FDA | 98 | 2 | 0 |
| Ex. 4.4 | Ex. 1.16 | 0.0025 | benzopurpurin 4B | 98 | 2 | 0 |
| Ex. 4.5 | Ex. 1.15 | 1.0 | Direct Red 83.1 | 99.6 | 0.4 | 0 |
| Ex. 4.6 | Ex. 1.15 | 1.1 | Brilliant Blue R | 99.9 | 0.1 | 0 |
| Ex. 4.7 | Ex. 1.15 | 1.0 | Sicopal Orange L2430 | 99.5 | 0.5 | 0 |
| Ex. 4.8 | Ex. 1.12 | 0.79 | Direct Red 83.1 | 99.5 | 0.5 | 0 |
| Ex. 4.9 | Ex. 1.12 | 0.97 | Direct Red 83.1 | 58.51 | 0.29 | 41.2 |
| Ex. 4.10 | Ex. 1.12 | 1.70 | Direct Red 83.1 | 8.66 | 0.04 | 91.3 |
| Ex. 4.11 | Ex. 1.12 | 0.91 | Sicopal Orange L2430 | 99.5 | 0.5 | 0 |
| Ex. 4.12 | Ex. 1.12 | 0.90 | Sicopal Orange L2430 | 63.58 | 0.32 | 36.1 |
| Ex. 4.13 | Ex. 1.12 | 1.79 | Sicopal Orange L2430 | 8.76 | 0.04 | 91.2 |
| Ex. 4.14 | Ex. 1.12 | 1.04 | Brilliant Blue R | 99.5 | 0.5 | 0 |
| Ex. 4.15 | Ex. 1.12 | 1.07 | Brilliant Blue R | 70.84 | 0.36 | 28.8 |
| Ex. 4.16 | Ex. 1.12 | 1.97 | Brilliant Blue R | 10.35 | 0.05 | 89.6 |

Table 6: Overview of beads containing dyes and different amounts of oily ingredient:

| Example | Description | Active Content (Cetiol OE) [wt.%] | Active Content (Cellulose) [wt.%] | Cellulose content Solution [wt.%] | Average Diameter [mm] | Average Force 50% Way [N] |
|---|---|---|---|---|---|---|
| Ex. 4.8 | Direct Red 83.1 Ethanol getrocknet | 0.0 | 100 | 6.0 | 0.787 | 56.89 |
| Ex. 4.9 | Direct Red 83.1 Ethanol/Cetiol OE getrocknet | 41.2 | 58.8 | 6.0 | 0.965 | 46.89 |
| Ex. 4.10 | Direct Red 83.1 Cetiol OE getrocknet | 91.3 | 8.7 | 6.0 | 1.696 | 13.36 |
| Ex. 4.11 | Sicopal Orange L 2430 Ethanol getrocknet | 0.0 | 100 | 6.0 | 0.911 | 59.35 |
| Ex. 4.12 | Sicopal Orange L 2430 Ethanol/Cetiol OE getrocknet | 36.1 | 63.9 | 6.0 | 0.900 | 42.30 |

(continued)

| Example | Description | Active Content (Cetiol OE) [wt.%] | Active Content (Cellulose) [wt.%] | Cellulose content Solution [wt.%] | Average Diameter [mm] | Average Force 50% Way [N] |
|---|---|---|---|---|---|---|
| Ex. 4.13 | Sicopal Orange L 2430 Cetiol OE getrocknet | 91.2 | 8.8 | 6.0 | 1.787 | 12.86 |
| Ex. 4.14 | Brilliant Blue R Ethanol getrocknet | 0.0 | 100 | 6.0 | 1.043 | 64.00 |
| Ex. 4.15 | Brilliant Blue R Ethanol/Cetiol OE getrocknet | 28.8 | 71.2 | 6.0 | 1.068 | 53.47 |
| Ex. 4.16 | Brilliant Blue R Cetiol OE getrocknet | 89.6 | 10.4 | 6.0 | 1.966 | 17.51 |

**List of Puricolor® -Types for Cosmetic and Personal Care Use**

**(BTC-Europe GmbH, Monheim am Rhein, Germany)**

Puricolor® Blue PBL29 FDA
(old: Ultramarine Blue 54) 77007
Pigment Blue 29
Ultramarine
Gunjou
Puricolor® Blue PBL15-L
(old: Vibracolor® Blue PBL15-L) 74160
Pigment Blue 15
Puricolor® Blue PBL 15:3
(old: Sicomet® Blue P 74160) 74160
Pigment Blue 15:3
Puricolor® Blue ABL9 FDA
(old: Puricolor® Blue ABL9) 42090
Acid Blue 9
FD&C Blue No 1
E133/Ao1
Puricolor® Blue FBL5
(old: Vibracolor® Blue FBL5) 42051
Food Blue 5
E131
Puricolor® Green PGR7-L
(old: Vibracolor® Green PGR7-L) 74260
Pigment Green 7
Puricolor® Green PGR7
(old: Vibracolor® Green PGR7) 74260
Pigment Green 7
Puricolor® Green SGR7 FDA
(old: Puricolor® Green SGR7) 59040
Solvent Green7
D&C Green No 8
Midori204
Puricolor® Green SGR7
(old: Vibracolor® Green SGR7) 59040
Solvent Green7
Puricolor® Green U3 FDA

(old: Puricolor® Green U3)

Puricolor® Yellow FYE13

(old: Vibracolor® Yellow FYE13) 47005

Food Yellow 13

E104

Ki203

Puricolor® Yellow PYE1-L

(old: Vibracolor® Yellow PYE1-L) 11680

Pigment Yellow 1

Puricolor® Yellow AYE17

(old: Vibracolor® Yellow AYE17) 18965

Acid Yellow 17

Puricolor® Yellow PYE13-L

(old: Vibracolor® Yellow PYE13-L) 21100

Pigment Yellow 13

Direct Yellow 28

Puricolor® Yellow AYE23 FDA

(old: Puricolor® Yellow AYE23) 19140

Acid Yellow 23

FD&C Yellow No 5

E102 / Ki4

Puricolor® Yellow PYE42 FDA

(old: Puricolor® Yellow PYE42) 77492

Pigment Yellow 42

synthetic iron oxide

E172 / Sankatetu

Puricolor® Yellow FYE3 FDA

(old: Puricolor® Yellow FYE3) 15985

Food Yellow 3

FD&C No 6

E110 / Ki5

Puricolor® Orange AOR7 FDA

(old: Puricolor® Orange AOR7) 15510

Acid Orange 7

D&C Orange No 4

Daidai205

Puricolor® Red FRE1 FDA

(old: Puricolor® Red FRE1) 14700

Food Red 1

FD&C Red No 4

Aka504

Puricolor® Red ARE18

(old: Vibracolor® Red ARE18)

Puricolor® Red FRE14

(old: Erythrosin 88 E 127) 45430

Food Red14

E127

Puricolor® Red ARE14

(old: Vibracolor® Red ARE14) 14720

Acid Red 14

E122

Puricolor® Red PRE5-L

(old: Vibracolor® Red PRE5-L) 12490

Pigment Red 5

Puricolor® Red ARE33 FDA

(old: Puricolor® Red ARE33) 17200

Acid Red 33

D&C Red No 33

Aka227

Puricolor® Red ARE52
(old: Vibracolor® Red ARE52) 45100
Acid Red 52

Aka106

Puricolor® Black PBLK7-L
(old: Vibracolor® Black PBK7-L) 77266
Pigment Black 7

**Claims**

1. Spherical beads with a diameter D(4,3) of 1 - 4000 $\mu$m comprising
   1 to 99.9 % by weight of amorphous cellulose,
   0.01 to 20 % by weight of dyes or pigments,
   **characterized in that** the weight ratio of dyes and pigments to cellulose is between 0.001/99.999 and 20/80.

2. Beads according to claim 1, **characterized in that** the dyes are selected from the group consisting of

   a) the oil soluble dyes Alizarine Cyanine Green, Ponceau G, Uranine, Tetra Bromo Fluoroscein, Tetra Bromo Fluoroscein (W), Phloxine B, Quinoline Yellow S. S., Alizarine Purple, Quinizarine Green,
   b) the water soluble dyes Acid Orange 7, Acid Fuchsine, Acid Fuchsine, Phloxine B [Na Salt], Eosine [Na Salt], Quinoline Yellow W. S., Alizarine Purple Ext, Uranine (Na Salt), Fast Green FCF, Ponceau SX, Sunset Yellow, E-110, Allura Red, E-129, Tartrazine, E-102, Brilliant Blue, E-133, Erythrosine, E-127, Indigo Carmine, E-132, Ponceau 4R, E-124, Acid Red 52, Carmoisine, E-122, Amaranth, E-123, Brown HT, Black PN. Green S, Patent Blue V, Tartrazine, Quinolline Yellow, Erythrosine, Allura Red, Brilliant Blue and
   c) Puricolor®-Types Puricolor® Red ARE33 FDA (Acid Red 33 (17200)), Puricolor® Yellow AYE23 FDA (Acid Yellow 23 (CI 19140)), Puricolor® Green SGR7 FDA (Solvent Green7 (CI 59040)), Puricolor® Red FRE1 FDA (Food Red 1 (CI 14700)), Puricolor® Green U3 FDA (Food Green 3 (CI 42053)), Puricolor® Yellow FYE13 (Food Yellow 13 (CI 47005)).

3. Beads according to claim 1 or 2, **characterized in that** the pigments are selected from the group consisting of chlorophyll, titanium dioxide, zinc oxide, iron oxides such as red iron oxide, yellow iron oxide, brown iron oxide and black iron oxide; chromium oxides, chromium hydroxides, ultramarine blue, indigo pigments, manganese ammonium pyrophosphates, manganese violet, cerium oxide, copper phtalocyanines and mixtures thereof.

4. Beads according to one of claims 1 to 3, **characterized in that** the beads comprise 0.1 to 98 % by weight of an oily ingredient.

5. Beads according to one of claims 1 to 4, **characterized in that** they comprise less than 10 % , preferably less than 5 % and more preferably less than 3 % by weight of water based on the weight of the beads.

6. Beads according to one of claims 1 to 5, **characterised in that** the weight ratio of dyes and pigments to cellulose is 0.01/99.99 to 10/90, preferably 0.01/99.99 to 5/95, more preferably it is 0.1/99.9 to 2/98, and most preferably it is 0.5/99.5 to 1.5/98.5.

7. Beads according to one of claims 1 to 6, **characterized in that** they comprise at least 50 % by weight, preferably at least 90 % by weight, more preferably at least 95 % by weight and most preferably at least 98 % by weight of amorphous cellulose based on the weight of the beads.

8. Beads according to one of claims 1, 2, 4 to 7, **characterized in that** they comprise dyes and are translucent.

9. Beads according to one of claims 1 to 6 and 8, **characterized in that** they contain oils and/or emollients and that their deformation between two parallel plates obeys to the rules
   F50 < 0.03 x D -14.95 for beads of D(4,3) of 500 to 1500 $\mu$m and
   F50 < 0.12 x D -149.95 for beads of D(4,3) of 1500 to 3000 $\mu$m
   with $F_{50}$ being the force measured in N that is necessary to compress the bead to 50% of its original diameter D measured in $\mu$m for beads containing more than 50 % by weight of oily ingredients based on the weight of the beads.

10. Beads according to one of claims 1 to 9, **characterized in that** they have a diameter D(4,3) of 10 to 3000 μm, preferably 100 to 2500 μm, more preferably 500 to 2000 μm determined by light microscopy.

11. Beads according to claims 1 to 10, **characterized in that** they comprise
    97 - 99.9 % by weight amorphous cellulose and
    0.1 - 3 % by weight of a dye or pigment
    based on the weight of the beads.

12. Beads according to claims 1 to 6, **characterized in that** they comprise
    1 - 20 % by weight amorphous cellulose and
    3 - 98 % by weight of an oily ingredient and
    0.01 to 10 % by weight of a dye or pigment
    based on the weight of the beads.

13. Cosmetic compositions comprising 0.1 to 50 wt % of the beads according to one of claims 1 to 12 based on the weight of the composition.

14. Use of the beads according to one of claims 1 to 12 as cosmetic scrubbing or peeling agent.

15. Use of the beads according to one of claims 1 to 12 for application of oils and emollients on the skin.

Figure 1:

Figure 2:

Figure 3:

Figure 4:

Europäisches Patentamt

European Patent Office

Office européen des brevets

# EUROPEAN SEARCH REPORT

Application Number

EP 19 21 2217

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2009/074359 A2 (UNILEVER PLC [GB]; UNILEVER NV [NL] ET AL.) 18 June 2009 (2009-06-18) | 1,5,13 | INV. A61K8/02 A61K8/73 A61Q19/00 |
| Y | * pages 3,11; claim 1 * | 1-15 | |
| Y | CN 107 550 742 A (WUHAN HUALI BIOMATERIAL CO LTD) 9 January 2018 (2018-01-09) * the whole document * | 1-15 | |
| Y | WO 2011/006658 A1 (CLARIANT INT LTD [CH]; LACHMANN ANGELA [DE] ET AL.) 20 January 2011 (2011-01-20) * pages 10-12 * | 1-15 | |
| Y,D | US 2004/131690 A1 (LYNCH MAURICE GERARD [BE]) 8 July 2004 (2004-07-08) * page 3 - page 5 * | 1-15 | |
| Y | JP 2004 331918 A (ASAHI KASEI CHEMICALS CORP) 25 November 2004 (2004-11-25) * the whole document * | 1-15 | |
| Y,D | MARTIN GERICKE ET AL: "Functional Cellulose Beads: Preparation, Characterization, and Applications", CHEMICAL REVIEWS, AMERICAN CHEMICAL SOCIETY, US , vol. 113, no. 7 29 March 2013 (2013-03-29), pages 4812-4836, XP002739481, ISSN: 0009-2665, DOI: 10.1021/CR300242J Retrieved from the Internet: URL:http://pubs.acs.org/doi/ipdf/10.1021/cr300242j [retrieved on 2015-05-12] * page 4813 ff * | 1-15 | TECHNICAL FIELDS SEARCHED (IPC) A61K A61Q |

-/--

The present search report has been drawn up for all claims

| Place of search | Date of completion of the search | Examiner |
|---|---|---|
| Munich | 25 March 2020 | Bader, Karl Günther |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 19 21 2217

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | Diana Ciolacu ET AL: "AMORPHOUS CELLULOSE - STRUCTURE AND CHARACTERIZATION", CELLULOSE CHEMISTRY AND TECHNOLOGY Cellulose Chem. Technol, 1 January 2011 (2011-01-01), pages 13-21, XP055679196, Retrieved from the Internet: URL:http://cellulosechemtechnol.ro/pdf/CCT1-2(2011)/p.13-21.pdf [retrieved on 2020-03-24] * the whole document * ----- | 1-15 | |

TECHNICAL FIELDS
SEARCHED      (IPC)

The present search report has been drawn up for all claims

| Place of search | Date of completion of the search | Examiner |
|---|---|---|
| Munich | 25 March 2020 | Bader, Karl Günther |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

page 2 of 2

**EP 3 827 806 A1**

## ANNEX TO THE EUROPEAN SEARCH REPORT
## ON EUROPEAN PATENT APPLICATION NO.

EP 19 21 2217

This annex lists the patent family members relating to the patent documents cited in the above-mentioned European search report.
The members are as contained in the European Patent Office EDP file on
The European Patent Office is in no way liable for these particulars which are merely given for the purpose of information.

25-03-2020

| Patent document cited in search report | | Publication date | Patent family member(s) | | Publication date |
|---|---|---|---|---|---|
| WO 2009074359 | A2 | 18-06-2009 | AR | 069177 A1 | 06-01-2010 |
| | | | AU | 2008334841 A1 | 18-06-2009 |
| | | | CA | 2707803 A1 | 18-06-2009 |
| | | | CN | 101896157 A | 24-11-2010 |
| | | | EP | 2217199 A2 | 18-08-2010 |
| | | | ES | 2586027 T3 | 11-10-2016 |
| | | | US | 2009155322 A1 | 18-06-2009 |
| | | | WO | 2009074359 A2 | 18-06-2009 |
| | | | ZA | 201003287 B | 31-08-2011 |
| CN 107550742 | A | 09-01-2018 | NONE | | |
| WO 2011006658 | A1 | 20-01-2011 | BR | 112012001088 A2 | 23-02-2016 |
| | | | CN | 102449130 A | 09-05-2012 |
| | | | EP | 2277982 A1 | 26-01-2011 |
| | | | EP | 2454354 A1 | 23-05-2012 |
| | | | EP | 2454355 A1 | 23-05-2012 |
| | | | ES | 2378495 T3 | 13-04-2012 |
| | | | JP | 5669836 B2 | 18-02-2015 |
| | | | JP | 5743229 B2 | 01-07-2015 |
| | | | JP | 2012533528 A | 27-12-2012 |
| | | | JP | 2012533637 A | 27-12-2012 |
| | | | KR | 20120052313 A | 23-05-2012 |
| | | | US | 2012178662 A1 | 12-07-2012 |
| | | | US | 2012183479 A1 | 19-07-2012 |
| | | | WO | 2011006657 A1 | 20-01-2011 |
| | | | WO | 2011006658 A1 | 20-01-2011 |
| US 2004131690 | A1 | 08-07-2004 | NONE | | |
| JP 2004331918 | A | 25-11-2004 | NONE | | |

EPO FORM P0459

For more details about this annex : see Official Journal of the European Patent Office, No. 12/82

30

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010019164 A
- EP 2190882 A
- US 20040131690 A
- WO 2019081246 A

### Non-patent literature cited in the description

- **XIAOGANG LUO et al.** *Journal of Materials Chemistry,* 2009, vol. 19, 3538-3545
- High effective adsorption of organic dyes on magnetic cellulose beads entrapping activated carbon. *Journal of Hazardous Materials,* 2009, vol. 171, 340-347
- Encapsulation of Either Hydrophilic or Hydrophobic Substances in Spongy Cellulose Particles. *American Chemical Society - ACS Appl. Mater. Interfaces,* 2017, vol. 9, 944-949
- **TARO OMURA et al.** *ACS Appl. Mater. Interfaces,* 2017, vol. 9, 944-949
- **PARKS et al.** *Biotechnology for Biofuels,* 2010, vol. 3 (10), 3-10
- Kosmetische Färbemittel. Farbstoffkommission der Deutschen Forschungsgemeinschaft. Verlag Chemie, 1984, 81-106